Europäisches Patentamt

European Patent Office

Office européen des brevets

(11) Numéro de publication: **0 263 025 B1**

(12) **FASCICULE DE BREVET EUROPEEN**

(45) Date de publication de fascicule du brevet: **30.06.93**  (51) Int. Cl.⁵: **A61K 39/245**, C07K 3/02, C07K 3/12, G01N 33/571

(21) Numéro de dépôt: **87402162.9**

(22) Date de dépôt: **29.09.87**

(54) Fractions contenant des antigènes spécifiques du virus de l'herpès type 1 (HSV-1) et type 2 (HSV-2), procédé d'isolement de ces fractions et méthode de diagnostic spécifique du virus de l'herpès HSV-1 et/ou HSV-2 à l'aide desdites fractions.

(30) Priorité: **30.09.86 GR 862472**

(43) Date de publication de la demande: **06.04.88 Bulletin 88/14**

(45) Mention de la délivrance du brevet: **30.06.93 Bulletin 93/26**

(84) Etats contractants désignés: **AT BE CH DE ES FR GB GR IT LI LU NL SE**

(56) Documents cités:
EP-A- 0 139 417
WO-A-83/02987
US-A- 4 341 754
US-A- 4 374 127
US-A- 4 430 437

CELLULAR & MOLECULAR BIOLOGY, vol. 25, 1980, Pergamon Press Ltd. (GB); G.TARRO et al., pp. 329-333&NUM;

(73) Titulaire: **INSTITUT PASTEUR HELLENIOUE 127, Avenue Vassilissis Sofias GR-115 21 Athenes(GR)**

(72) Inventeur: **Markoulatos, Panayotis 7, Rue Karaoli-Dimitriou Nea Helvetia Athenes 16232(GR)**

(74) Mandataire: **Ores, Irène et al CABINET ORES 6, Avenue de Messine F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3. 10 / 3.6 / 3.3. 1)

**Description**

La présente invention est relative à l'obtention de fractions contenant des antigènes spécifiques du virus de l'herpès type 1 (HSV-1) et type 2 (HSV-2), et plus particulièrement de la glycoprotéine gC de HSV-1 et de la glycoprotéine gD de HSV-2, ainsi qu'à l'utilisation de ces fractions pour le diagnostic différencié d'infections par HSV-1 et par HSV-2.

Les types 1 (HSV-1) et 2 (HSV-2) du virus de l'herpès peuvent être différenciés par leur profil antigénique (Dowdle et al, 1967, Journal of Immunology, 99, p.974-980), et par leur composition génétique (Ludwig et al, 1972, Virology, 49, p.95-101). Lors d'une infection par le virus de l'herpès, HSV, les polypeptides structuraux du virus sont incorporés dans les membranes cellulaires (Norrild et al, 1979, Journal of Virology, 32, p.741-748) ; parmi ces polypeptides, les glycoprotéines spécifiques du virus vont former des cibles pour les réponses immunes humorales et cytotoxiques (Norrild, 1980, Current Topics in Microbiology and Immunology, 90, p.67-106). La détermination sérologique des virus de types 1 et 2, responsables de la réponse immunologique, peut être basée sur la détection d'anticorps dirigés contre une ou plusieurs de ces glycoprotéines. Néanmoins, les anticorps qui se développent contre les virus HSV-1 ou HSV-2 présentent une réactivité croisée intertypique très grande (Hampar et Martos, 1973, Immunological relationships, p.221-259 in AS Kaplan, The Herpes viruses). Cette forte réactivité croisée entre les virus HSV-1 et HSV-2 constitue le principal problème dans le sérodiagnostic des infections par le virus de l'herpès, ce problème étant dû à la difficulté d'obtenir des antigènes viraux présentant un degré de spécificité suffisant pour permettre un diagnostic différencié.

Or, il est indispensable de pouvoir établir un tel diagnostic, notamment par exemple en ce qui concerne la pathologie la plus grave due au virus herpétique, à savoir l'encéphalomyélite, pour laquelle le virus de l'herpès type 1 a été identifié comme antigène responsable de cette maladie.

De nombreux tests sérologiques ont été décrits dans l'Art antérieur, en vue de distinguer les anticorps anti-HSV-1 des anticorps anti-HSV-2 dans le sérum humain. On peut ainsi citer les tests cinétiques de microneutralisation (Pauls and Dowdle, 1967, Journal of Immunology 98, p.941-947) et de neutralisation (Rawls et al, 1968, Herpes-virus type 2:association with carcinoma of the cervix, Science 161, p.1255-1256), l'hemagglutination indirecte (Fucilo et al, 1970, Proceedings of Society of Experimental Biology and Medicine 133, p. 735-739), les radioimmuno-essais en phase solide (Forganni et al, 1975, Journal of Clinical Micro-biology 2, p.410-418), les radioimmuno-essais avec des protéines virales purifiées (Heilman et al, 1979, Journal of Virology 29, p.34-42), l'immunoélectrophorèse (Vestergaard, 1979, Infection and Immunity 23, p.553-558) et les méthodes immunoenzymatiques comme la technique ELISA (Vestergaard et Grauballe, 1979, Acta Pathologica Microbiologica Scandinavia, Section B 87 : p.261-263 ; Vestergaard, 1982, Developments in Biological Standardization 52, p.95-98). Mais ces tests ne permettent pas de distinguer nettement entre les infections par les virus HSV-1 et celles par les virus HSV-2, essentiellement par manque d'antigènes spécifiques du type, suffisamment purifiés. Il est donc important de pouvoir identifier de façon différenciée les virus des types HSV-1 et HSV-2 respectivement, responsables des deux types d'infections par HSV.

Les glycoprotéines gC et gD sont fortement immunogènes et constituent les antigènes cibles présents dans les enveloppes virales et à la surface des cellules infectées (Norrild, 1980, Current Topics in Microbiology and Immunology 90,p.67-106). Bien que l'on ait pensé que la glycoprotéine gC de HSV-1 possédait seulement des épitopes spécifiques du sérotype, il a été montré récemment que cette glycoprotéine peut aussi avoir des déterminants antigéniques responsables de réactions croisées (Zweig et al, 1983, Journal of Virology 47, p. 185-192 ; Zesulak et al, 1983, Journal of Virology 47, p. 553-562). Au vu de l'existence de ces déterminants antigéniques communs à HSV-1 et HSV-2, il a d'ailleurs été proposé d'utiliser des préparations de gC ou gD pour l'obtention de vaccins assurant une protection contre les deux types de virus. L'utilisation d'anticorps monoclonaux a mis en évidence que plusieurs polypeptides de HSV-1 et HSV-2, y compris les glycoprotéines gC et gD, expriment des déterminants antigéniques à réactivité croisée, aussi bien que des déterminants antigéniques spécifiques du type (Pereira et la, 1980, Infection and Immunity 29, p. 724-732 ; Showalter et al, 1981, Infection and Immunity 34, p. 684-692 ; Pereira et al, 1982, Infection and Immunity 35, p. 363-367 ; Zweig et al, 1983, Journal of Virology 47, p. 185-192) ; Demande PCT 83/02897, qui décrit l'utilisation d'un même anticorps monoclonal pour purifier, par immunoaffinité, la glycoprotéine gD de HSV-1 et la glycoprotéine gD de HSV-2.

Arvin et al (1983, Infection and Immunity 40, p. 184-189), en utilisant la glycoprotéine gC de HSV-1 purifiée par immunoaffinité, Hampar et al (1983, Journal of Clinical Microbioiogy 21, p.496-500), en utilisant les glycoprotéines gC et gD de HSV-1 et gD et gF de HSV-2 purifiées par immunoaffininité également, de même que Svennerholm et al (1984, Journal of Clinical Microbiology, 19, pages 235-239) Suchankova et al (1984, Journal of Infectious Diseases 149, p. 964-971), en utilisant des antigènes de HSV-1 (glycoprotéine gC) et de HSV-2 (polypeptide de 130 000 daltons), purifiés par la lectine de Helix pomatia, ont développé des tests immunoenzymatiques (ELISA) ou des radioimmuno-essais avec ces différentes protéines pour la détection d'anticorps anti-HSV-1 et/ou anti-HSV-2. Bien que des protéines individuelles puissent, en principe, être utilisées comme antigène cible, des précautions particulières devraient être prises en cas d'utilisation de tels antigènes, car la variation anti-génique observée d'une souche de virus herpétique à une autre peut conduire à des taux d'anticorps modifiés en réponse à des antigènes individuels. Ceci pourrait réduire la sensibilité des test jusqu'à des niveaux trop faibles pour être interprétables et conduire à des diagnostics imprécis.

En outre, toutes ces fractions contenant des antigènes spécifiques de type ont été préparées par des méthodes immunologiques ou par des procédés de chromatographie par affinité ; ces procédés sont longs à mettre en oeuvre, coûtent cher (exemple : la lectine d'Helix pomatia) ou dépendent de la disponibilité d'anticorps monoclonaux (exemple : les méthodes par immunoaffinité).

La Demande de Brevet Européen (EP 0 183 215) propose une méthode pour la détermination d'anticorps anti-virus herpétique par un test d'agglutination passive, mais cette dernière est incapable de distinguer entre les infections par HSV-1 ou par HSV-2 et la sensibilité du test est en outre très faible.

La technique ELISA est une méthode immunoenzymatique de titrage d'anticorps : un antigène fixé sur un support solide se lie avec l'anticorps spécifique contenu dans le milieu biologique à tester et l'immun-complexe obtenu est détecté par un anticorps anti-espèce marqué par une enzyme ; on ajoute ensuite un substrat spécifique de l'enzyme, dont la dégradation par l'enzyme fait apparaître un produit coloré détecté d'une manière appropriée.

McEwen (Analytical Biochemistry, vol. 20, n°1, Juillet 1967, p. 114-149) a mis au point une méthode de calcul de la vitesse de sédimentation de particules dans des gradients de densité de saccharose, dans laquelle il calcule le coefficient de sédimentation d'une particule dans un gradient linéaire de saccharose, à l'aide de la formule

$$z_0 = \frac{z_1 r_2 - z_2 r_1}{r_2 - r_1}$$

dans laquelle $z_0$ est la concentration du soluté correspondant à l'extrapolation de la concentration du gradient linéaire du du soluté au rayon zéro, $z_1$ et $z_2$ sont respectivement les pourcentages maximum et minimum du gradient de saccharose du soluté exprimés en poids par rapport au poids total du soluté, $r_1$ et $r_2$ sont respectivement les distances radiales minimum (sommet du tube de centrifugation) et maximum (fond du tube de centrifugation) par rapport à l'axe de centrifugation ($r_1$ et $r_2$ sont spécifiques pour chaque type d'appareil employé). Le calcul de la concentration de saccharose à l'axe de centrifugation, quand r = 0, détermine le signe de la valeur de $z_0$, et généralement cette valeur est négative. La sédimentation d'une protéine à partir de la position initiale $z_y$ (minimum de pourcentage du gradient de saccharose) jusqu'à une nouvelle position $z_x$ (pourcentage de gradient de saccharose où la protéine est détectée à la fin de la centrifugation) dépend de la différence entre les deux valeurs $z_x$ et $z_y$, exprimées par deux valeurs correspondantes de "temps intégral" $I_2$ et $I_1$, calculées à l'aide des Tables de McEwen en fonction de la densité de la particule (en général on admet des densités de 1,3 à 1,4 pour la plupart des protéines), de la température et de la valeur de $z_0$. Le coefficient de sédimentation est alors calculé par la formule

$$S_{20,w} = \frac{\Delta I}{\omega^2 t}$$

où $\Delta I = I_2 - I_1$, où $\omega = 0{,}1047 \times$ tpm (tpm étant la vitesse de centrifugation) et où t est le temps exprimé en secondes.

La présente invention s'est en conséquence donné pour but de pourvoir à un procédé d'obtention de fractions contenant des antigènes spécifiques du virus de l'herpès type 1 (HSV-1) et type 2 (HSV-2), et plus particulièrement la glycoprotéine gC de HSV-1 et la glycoprotéine gD de HSV-2, lequel procédé répond mieux aux nécessités de la pratique que les procédés d'obtention de fractions antigéniques antérieurement connus, notamment en ce qu'il est spécifique, fiable, peu onéreux, et permet l'obtention de fractions antigéniques à degré de spécificité élevé, pour la mise en oeuvre d'un test de diagnostic différencié d'une infection par le virus de l'herpès, qui est apte à déterminer avec précision une infection par HSV-1 ou par HSV-2.

EP 0 263 025 B1

La présente invention a pour objet un procédé de purification de fractions antigéniques spécifiques de type de HSV-1 ou de HSV-2, dans lequel on procède à la multiplication in-vitro, sur des cellules de mammmifère approprié, de cultures de virus HSV-1 et/ou HSV-2, jusqu'à la lyse d'au moins une proportion importante de cellules, puis on lyse les cellules restantes par des agents chimiques appropriés, et après désagrégation de leur paroi par traitement physique, notamment par ultrasons, on les soumet à une centrifugation, et l'on centrifuge le surnageant obtenu sur un gradient linéaire de saccharose, lequel procédé est caractérisé en ce que les antigènes spécifiques de HSV-1 et de HSV-2 sont respectivement recueillis dans les fractions du gradient contenant de 20 à 27% en poids de saccharose, et dans les fractions du gradient contenant de 6 à 10% en poids de saccharose. L'invention a également pour objet une fraction antigénique purifiée spécifique de type de HSV-1, caractérisée en ce qu'elle est susceptible d'être obtenue par le procédé, ci-dessus dans les fractions de 20 à 27% en poids d'un gradient linéaire de saccharose, et en ce que son coefficient de sédimentation est compris entre 9,4 et 9,6 S, ainsi qu'une fraction antigénique purifiée spécifique de type de HSV-2, caractérisée en ce qu'elle est susceptible d'être obtenue par le procédé, ci-dessus dans les fractions de 6 à 10 % en poids d'un gradient linéaire de saccharose, et en ce que son coefficient de sédimentation est compris entre 4,3 et 4,5 S.

Selon une disposition avantageuse de ce mode de réalisation, la suspension de virus HSV-1 et/ou HSV 2 est elle-même obtenue à partir d'une culture de cellules de mammifères infectées par HSV-1 et HSV-2, centrifugée pour fournir une fraction soluble qui, après avoir été lysée et désagrégée, est soumise au processus susdit de centrifugation dans un gradient de saccharose.

Selon une autre disposition avantageuse de la présente invention, lesdites fractions antigéniques présentent une concentration en protéines de l'ordre de 200 à 300 $\mu$g/ml.

La présente invention a également pour objet un procédé pour l'isolement des fractions contenant les antigènes spécifiques HSV-1 et/ou HSV-2, définies dans ce qui précède, caractérisé en ce que l'on infecte des cellules d'un mammifère approprié, avec des cultures de virus HSV-1 et/ou HSV-2, dans lesquelles le virus se multiplie jusqu'à la lyse d'au moins une proportion importante des cellules, en ce que les cellules restantes sont lysées par traitement par des agents chimiques appropriés, puis leur paroi est désagrégée par traitement physique, notamment par ultrasons, et elles sont soumises à une centrifugation, en ce que la solution surnageante est centrifugée dans un gradient linéaire de saccharose ou d'un autre milieu approprié, et en ce que les fractions contenant les antigènes spécifiques HSV-1 et/ou HSV-2 sont récupérées par tous moyens appropriés.

Selon un mode de réalisation avantageux du procédé conforme à la présente invention, les cultures de virus englobent aussi bien les suspensions virales obtenues après lyse totale des cellules, que les cultures de cellules contenant le virus, et qu'un mélange de cellules infectées et de virus libéré des cellules lysées.

Selon un mode de réalisation avantageux du procédé conforme à la présente invention, les polypeptides doués de propriétés antigéniques HSV-1 et/ou HSV-2 contenus dans les fractions antigéniques susdites sont isolés sur un gradient linéaire de saccharose ou d'un autre milieu approprié, déterminé en fonction de leur coefficient de sédimentation calculé.

Selon une disposition avantageuse de ce mode de réalisation, le coefficient de sédimentation est calculé d'après la formule

$$s_{20,w} = \frac{\Delta I}{\omega^2 t}$$

dans laquelle $\Delta I$ représente la différence entre deux valeurs de sédimentation intégrale extrêmes estimées en fonction de la densité de la protéine, de la température et de la concentration du soluté de saccharose, dans laquelle $\omega$ représente la vitesse de centrifugation et dans laquelle t représente le temps.

Selon une modalité avantageuse de cette disposition, le coefficient de sédimentation calculé est compris entre 9 et 9,6 S pour la glycoprotéine gC.

Selon une autre modalité avantageuse de cette disposition, le coefficient de sédimentation calculé est compris entre 4,3 et 4,5 S pour la glycoprotéine gD.

Selon un mode de réalisation avantageux du procédé conforme à la présente invention, le gradient linéaire de saccharose pour l'isolement des fractions spécifiques HSV-1 et /ou HSV-2 est réalisé à des concentrations de saccharose comprises entre 6 et 27 % du poids total de la solution de saccharose.

Selon une disposition avantageuse de ce mode de réalisation, la concentration de saccharose nécessaire pour isoler la glycoprotéine gC de HSV-1 est comprise préférentiellement entre 10 et 27 % du poids total de la solution de saccharose.

4

Selon une autre disposition avantageuse de ce mode de réalisation, la concentration de saccharose nécessaire pour isoler la glycoprotéine gD de HSV-2 est comprise préférentiellement entre 6 et 19 % du poids total de la solution de saccharose.

Selon un mode de réalisation avantageux du procédé conforme à la présente invention, les fractions contenant les antigènes spécifiques HSV-1 et/ou HSV-2 sont identifiées séparément par électrophorèse et immunotransfert, chaque fraction étant respectivement recueillie dans la fraction de soluté contenant de 6 à 10 % de saccharose par rapport au poids total de la solution de saccharose pour le polypeptide de 46 000 daltons, et dans la fraction de soluté contenant de 20 à 27 % de saccharose par rapport au poids total de la solution de saccharose pour le polypeptide de 125 000 daltons.

La présente invention a en outre pour objet une méthode de diagnostic in vitro des infections par le virus de l'herpès des types 1 ou 2, par détermination d'anticorps anti-HSV-1 et/ou anti-HSV-2 dans un milieu biologique, caractérisée en ce que l'on met en contact des fractions contenant les antigènes spécifiques HSV-1 et/ou HSV-2 conformes à la présente invention, fixées d'une manière appropriée sur un support solide approprié, avec le milieu biologique présumé contenir des anticorps anti-HSV-1 et/ou anti-HSV-2 examiné, en ce que l'immun-complexe obtenu dans le cas où des anticorps anti-HSV-1 et/ou anti-HSV-2 sont effectivement présents, est détecté par un anticorps anti-espèce marqué par une enzyme, en présence d'un substrat spécifique de l'enzyme.

Selon un mode de réalisation avantageux de la méthode de diagnostic conforme à la présente invention, les fractions contenant les antigènes spécifiques HSV-1 et/ou HSV-2 sont fixées sur ledit support à des dilutions avantageusement comprises entre 0,375 et 6 $\mu$g de protéines par ml.

Selon un autre mode de réalisation avantageux de la méthode de diagnostic conforme à la présente invention, le support solide est choisi dans le groupe des supports comprenant notamment les microplaques, les tubes, les globules rouges, les billes de polystyrène et les feuilles de nitrocellulose.

La présente invention a également pour objet un ensemble, ou kit, de diagnostic, pour la détermination, dans des liquides biologiques corporels, d'anticorps anti-HSV-1 et/ou anti-HSV-2, qui comprend comme composants principaux :
- une quantité appropriée, éventuellement subdivisée en doses unitaires, de différentes dilutions de fractions conformes à la présente invention contenant les antigènes spécifiques HSV-1 et de fractions contenant les antigènes spécifiques HSV-2 ;
- une quantité appropriée, éventuellement subdivisée en doses unitaires, suffisante pour la détermination précédente, d'anticorps anti-espèce marqués par une enzyme, pour la révélation de la réaction antigène-anticorps entre l'anticorps à déterminer et l'antigène spécifique correspondant
- éventuellement une quantité appropriée de tampons, diluants, réactifs, nécessaires à la mise en oeuvre de la méthode de diagnostic conforme à la présente invention.

L'invention sera mieux comprise à l'aide du complément de description qui va suivre, qui se réfère aux dessins annexés et à des exemples de mise en oeuvre du procédé objet de la présente invention.

Il doit être bien entendu, toutefois, que ces dessins, parties descriptives correspondantes et exemples de mise en oeuvre sont donnés uniquement à titre d'illustration de l'objet de l'invention, dont ils ne constituent en aucune manière une limitation.

**EXEMPLE 1 :** Préparation des antigènes spécifiques des types

Cellules et virus

- Des cellules primaires de rein de lapin (RL) sont préparées à partir de lapins âgés de 21 jours par des méthodes connues (Kuchler,1977,Biochemical methods in cell culture and Virology, p. 3-15). Les cellules sont transformées trois fois au maximum avant leur utilisation. Des cellules MRC-5 (ATCC,Rockville) sont cultivées comme les cellules RL dans du milieu Dulbecco (Laboratoires Flow) additionné de 10 % de sérum de veau foetal (Gibco), 100 $\mu$g de streptomycine/ml, 100 unités de pénicilline/ml et 0,2 % de bicarbonate de sodium.
- On injecte aux cellules RL, des souches de virus herpétique type 1 (HSV-1) et type 2 (HSV-2) (ATCC souches VR/733 et VR/734 respectivement) à une multiplicité d'infection (MOI) égale à 0,01 particule virale infectieuse par ml. Le stock viral est obtenu dans le liquide de culture extracellulaire lorsque le virus a lysé la totalité des cellules. Le liquide de culture est alors centrifugé à 1000 g pendant 10 minutes et le surnageant est réparti en aliquots de 2 ml et congelé à -70°C.

Préparation des antigènes

Les cellules monocouches RL lysées sont lavées des fois dans du PBS froid (137 mM de NaCl, 3 mM de KCl, 8 mM de $Na_2HPO_4$, 2 mM de $KH_2PO_4$, pH 7,4) contenant des inhibiteurs de protéases comme la tosyl-lysyl-chlorométhylcétone, le p-hydroxy mercuribenzoate et le fluorure de phénylméthylsulfonyle (Sigma Chemicals) à une concentration finale de 1 mM. Puis les cellules sont grattées dans de la glycine froide à 0,020 M et du tampon Tris à 0,0076 M (pH 8,6) contenant 3 % (vol./vol.) de Triton® X-100 et des inhibiteurs de protéases comme indiqués ci-dessus. Après un séjour de 30 minutes sur de la glace, les cellules lysées sont désagrégées par des ultrasons (18 kc/sec, deux fois pendant 15 secondes sur de la glace), puis centrifugées à 1000 g pendant 10 minutes. Les surnageants sont centrifugés à 100 000 g pendant une heure. Le surnageant des préparations d'antigène pour HSV-1 et HSV-2 et des cellules témoins sont échantillonnés et stockés à -70° C. La concentration en protéines dans les fractions solubles est déterminée par la méthode de Lowry modifiée (Journal of Biological Chemistry 192:265-275)) pour effectuer la mesure dans des solutions contenant du Triton® X-100 (Dulley et Grieve, 1975 Analytical Biochemistry 64:136-141) ; la concentration en protéines des préparations d'antigènes solubilisées est ajustée à 10 mg/ml.

Préparation des fractions contenant les antigènes spécifiques des types

Ces fractions solubles sont dialysées contre du tampon TNE (0,01 M de Tris-HCl, pH 7,4 ; 0,01 M de NaCl ; 0,001 M d'EDTA) pendant une nuit à +4° C. Puis 0,4 ml (4 mg) sont prélevés par tube, et ceci pour chaque préparation d'antigènes HSV-1, HSV-2 et des cellules témoins, et ils sont déposés sur 12,3 ml d'un gradient linéaire de saccharose préparé dans du tampon TNE. Pour la préparation des antigènes spécifiques HSV-1 et HSV 2, on réalise un gradient linéaire de saccharose de 10 à 27 % de saccharose par rapport au poids total du soluté pour HSV-1 et de 6 à 19 % de saccharose par rapport au poids du soluté pour HSV-2. Les gradients sont ensuite centrifugés pendant 24 heures (40 000 tours/minute, +5° C, rotor SW 41 Ti, Beckman) puis on recueille 0,4 ml de fractions contenant les antigènes spécifiques qui vont être soumises à une immunoélectrophorèse en fusée croisée avec un gel intermédiaire (Portocala et coll., 1983, Analysis of centrifugation gradients by fused rocket immunoelectrophoresis, 82, p.587-597).

**EXEMPLE 2 :** Immunoélectrophorèses en fusée avec un gel intermédiaire et avec du sérum de lapin hyperimmun anti-HSV-1 et anti-HSV-2.

- Préparation du sérum de lapin hyperimmun.

Des anticorps anti-HSV-1 et anti-HSV-2 polyspécifiques sont obtenus après infection de lapins par injections sous-cutanées successives de préparations d'antigènes (provenant des cellules RL infectées de l'exemple 1) mélangées à volume égal avec de l'adjuvant incomplet de Freund (2 x 0,1 ml) ; les injections se font une fois par mois pendant 12 mois. Les immunoglobulines de lapins sont ensuite purifiées (Harboe et Ingild, 1973, Scandinavian Journal of Immunology 2 (suppl.1):161-164) et leur concentration ajustée à 40 mg/l.

- Immunoélectrophorèse en fusée et en fusée croisée.

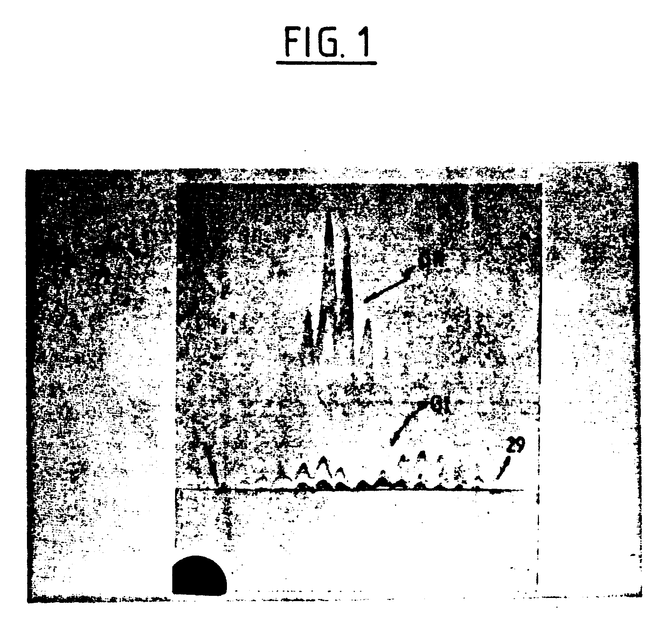

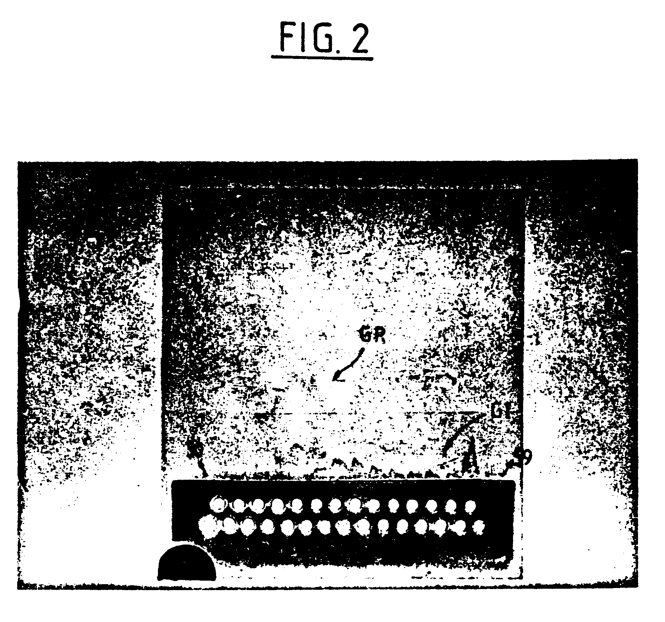

Les anticorps anti-HSV-1 et anti-HSV-2 sont disposés dans 1 % de gel d'agarose HSB (Litex, Danemark) avec du tampon Tris-tricine (9,7 g Tris, 4,3 g Tricine, 0,92 g de lactate de calcium, 0,2 g de $NaN_3$, pH 8,6) et 1 % (vol./vol.) de Triton® X-100. La quantité d'antigène solubilisé mise en oeuvre est de 7 $\mu$l pour l'immunoélectrophorèse en fusée et de 20 $\mu$l pour l'immunoélectrophorèse en fusée croisée. L'opération se déroule dans les conditions suivantes : 1,5 V/cm pendant 18 heures à 16° C (Axelsen, 1973, Scandinavian Journal of Immunology 2(suppl.1):p.71-77). Les résultats sont représentés aux Figures 1 et 2 annexées.

La Figure 1 montre une immunoélectrophorèse en fusée avec un gel intermédiaire d'une préparation d'antigènes HSV-1 fractionnée. 29 fractions ont été analysées, de la fraction 1 sur la gauche à la fraction 29 située à l'extrême droite. Les anticorps présents dans le gel intermédiaire $G_i$ proviennent de sérum de lapin hyperimmun vis-à-vis de HSV-2 (25 $\mu$l/cm²) et dans le gel de référence Gr, or trouve le sérum de lapin hyperimmun vis-à-vis de HSV-1 (10 $\mu$l/cm²). Le gel a été coloré par du Bleu brillant de Coomassie.

La Figure 2 montre une immunoélectrophorèse en fusée avec un gel intermédiaire d'une préparation d'antigènes HSV-2 fractionnée. 29 fractions ont été analysées, de la fraction 30 sur la gauche à la fraction 59 située à l'extrême droite. Les anticorps présents dans le gel intermédiaire $G_i$ proviennent de sérum de lapin hyperimmun vis-à-vis de HSV-1 (25 $\mu$l/cm²) et dans le gel de référence Gr, on trouve le sérum de lapin hyperimmun vis-à-vis de HSV-2 (10 $\mu$l/cm²). Le gel a été coloré par du Bleu brillant de Coomassie.

EP 0 263 025 B1

L'immunoélectrophorèse en fusée avec un gel intermédiaire d'antigènes HSV-1 ou HSV-2 révèle la distribution d'un antigène identifié dans le gel de référence (qui est superposé au gel intermédiaire) et le degré de contamination avec d'autres antigènes (gel intermédiaire) de ces fractions. Les préparations de cellules témoins ne donnent pas de pics dans le gel de référence. Les fractions qui montrent des pics dans le gel de référence sont les fractions contenant les antigènes spécifiques. La concentration en protéines de chaque fraction varie de 200 à 300 $\mu$g/ml.

**EXEMPLE 3 :** Calcul du coefficient de sédimentation des fractions contenant respectivement les antigènes spécifiques HSV-1 et les antigènes spécifiques HSV-2.

- On utilise la méthode de McEwen pour estimer le coefficient de sédimentation d'une particule dans un gradient linéaire de saccharose. On utilise les fractions détectées par immunoélectrophorèse en fusée dans l'exemple 2. Deux ou trois de ces fractions présentant les plus hauts pics sont recueillies pour chaque type d'antigène et leur concentration en saccharose est mesurée. Dans la formule

$$z_0 = \frac{z_1 r_2 - z_2 r_1}{r_2 - r_1}$$

les valeurs de $r_1$ et $r_2$ sont données par le constructeur de l'appareil : en l'occurrence, pour l'appareil utilisé (Beckman, L8-55, rotor SW 41 Ti), ces valeurs sont respectivement de 6,7 cm et 15,2 cm. Les conditions d'ultracentrifugation sont :
vitesse : 40 000 tours/minute,
durée : 24 heures,
température : $+5°C$.
- Calcul du coefficient de sédimentation de la fraction contenant l'antigène spécifique HSV-1.
Les valeurs de $Z_1$ et $Z_2$ sont respectivement de 10 et 27 %.
Dans la formule de McEwen, on trouve ainsi :

$$z_0 = \frac{z_1 r_2 - z_2 r_1}{r_2 - r_1} = \frac{(10 \times 15,2) - (27 \times 6,7)}{15,2 - 6,7} = -3,4$$

La valeur la plus proche de - 3,4 dans les Tables de McEwen est : - 5.
Pour calculer le coefficient de sédimentation, on utilise la formule

$$S_{20,w} = \frac{\Delta I}{\omega^2 t}$$

(une unité Svedberg S = $10^{-13}$ sec.)

$\omega^2$ = (0,10472 x tours/minute)$^2$ = (0,10472 x 40000)$^2$ = 1,75 x $10^7$ sec$^{-2}$
t = 24 heures = 86 400 sec.
$\omega^2 t$ = 1,75 x $10^7$ x 86,400 = 1,5 x $10^{12}$ sec$^{-1}$

Le Tableau I ci-dessous montre les valeurs de $I_2$ et $I_1$ calculées d'après les Tables de McEwen pour une valeur de $Z_x$ de 19 % et une valeur de $Z_y$ de 10 %, et le coefficient de sédimentation obtenu pour des densités de protéines égales à 1,3 et 1,4.

7

TABLEAU I

| Densité des protéines | 1,30 | 1,40 |
|---|---|---|
| $(Z_x)$ | 19 % (p/p) | 19 % (p/p) |
| $(Z_y)$ | 10 % (p/p) | 10 % (p/p) |
| $I_2$ (19 %) | 3,4490 | 3,3186 |
| $I_1$ (10 %) | 2,0058 | 1,9668 |
| $I = I_2 - I_1$ | 1,4432 | 1,3518 |
| $S_{20,w}$ | $9,6.10^{-13}$ sec = 9,6 S | $9,10^{-13}$ sec = 9 S |

- Calcul du coefficient de sédimentation de la fraction contenant l'antigène spécifique HSV-2.
Les valeurs de $Z_1$ et $Z_2$ sont respectivement de 6 et 19 %.

$$z_0 = \frac{z_1 r_2 - z_2 r_1}{r_2 - r_1} = \frac{(6 \times 15,2) - (19 \times 6,7)}{15,2 - 6,7} = -4,24$$

La valeur la plus proche de - 4,24 dans les Tables de McEwen est : - 5.
Pour calculer le coefficient de sédimentation, on utilise la formule

$$S_{20,w} = \frac{\Delta I}{\omega^2 t}$$

dans les conditions décrites ci-dessus.

Le Tableau II ci-dessous montre les valeurs $I_2$ et $I_1$ calculées d'après les Tables de McEwen pour une valeur de $Z_x$ de 10 % et une valeur de $Z_y$ de 6 %, et le coefficient de sédimentation obtenu pour des densités de protéines égales à 1,3 et 1,4.

TABLEAU II

| Densité des protéines | 1,30 | 1,40 |
|---|---|---|
| $(Z_x)$ | 10 % (p/p) | 10 % (p/p) |
| $(Z_y)$ | 6 % (p/p) | 6 % (p/p) |
| $I_2$ (10 %) | 2,0059 | 1,9668 |
| $I_1$ ( 6 %) | 1,3364 | 1,3192 |
| $\Delta I = I_2 - I_1$ | 0,6695 | 0,6476 |
| $S_{20,w}$ | $4,5.10^{-13}$ sec = 4,5 S | $4,3.10^{-13}$ sec = 4,3 S |

**EXEMPLE 4 :** Détermination du poids moléculaire des glycoprotéines gC de HSV-1 et gD de HSV-2.

- Marquage radioactif des polypeptides viraux.
Des monocouches confluentes de cellules RL sont infectées suivant la procédure de l'exemple 1 à une MOI de 5. Puis ces cellules sont marquées 4 à 20 heures après l'infection, soit avec 10 $\mu$Ci de $^{35}$S-méthionine par ml,(1,370 Ci/mmole) provenant d'un milieu contenant 0,05% en concentration normale de méthionine froide, soit avec 0,8 $\mu$Ci de D-(1-$^{14}$C)-glucosamine par ml (54 mCi/mmole) provenant d'un milieu contenant 10% de concentration usuelle en glucose (les radioisotopes proviennent de la Société Amersham, Angleterre).
- Séparation des immunoprécipités radioactifs du gel contenant les anticorps.
Les antigènes spécifiques de type marqués comme décrit ci-dessus, sont soumis à une immunoélectrophorèse en fusée croisée à travers un gel intermédiaire. On découpe et recueille 15 pics dans le gel de référence et les polypeptides contenus dans ces pics sont élués dans 400 $\mu$l d'un tampon Tris-$H_3PO_4$ 0,06 M (pH 6,9) contenant 2 % (en poids/vol.) de dodécylsulfate de sodium (SDS) et 2,5 % (vol./vol.) de 2-mercaptoéthanol (Norrild et coll., 1977, Analytical Biochemistry 81:432-441).

   - Electrophorèse analytique sur gel de polyacrylamide-SDS (SDS-PAGE).

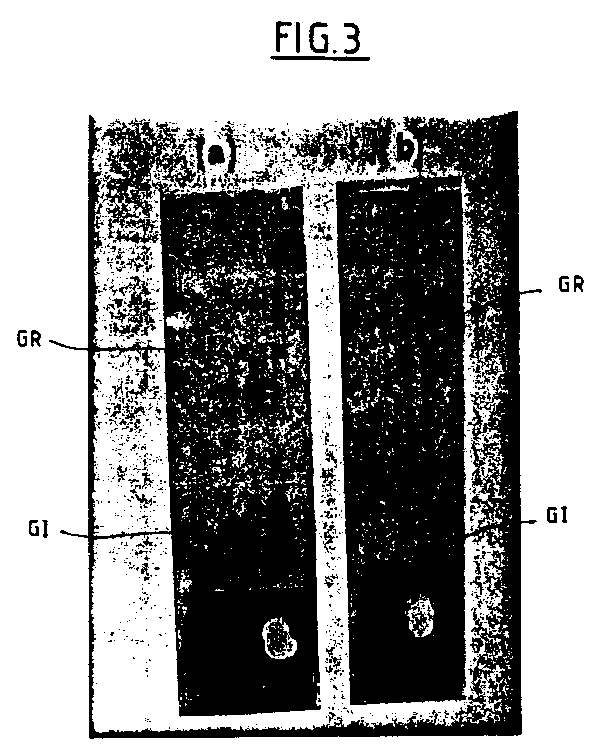

   Des plaques de gel utilisant le système de gel de polyacrylamide discontinu sont employées (Laemmli ; 1970, Nature 227:680-685). La concentration de polyacrylamide est de 7,5 % (poids/vol.) dans le gel de séparation et de 3,5 % dans le gel apparent. Les deux gels sont réticulés avec 0,2 % (poids/vol.) de N,N'-méthylènebisacrylamide. Les échantillons sont soumis à l'électrophorèse pendant 20 heures à 7 mA. Pour déterminer le poids moléculaire, on utilise comme marqueurs, des protéines marquées au carbone 14 (fournis par Amersham, Angleterre), telles que la myosine méthylée (PM 200 000), la méthylphosphorylase b (PM 100 000 et 92 500), la méthyl-sérumalbumine bovine (PM 69 200), la méthylovalbumine (PM 46 000), la méthyl-anhydrase carbonique (PM 30 000) et le méthyllysozyme (PM 14 300). Les protéines radiomarquées sont visualisées par autoradiographie effectuée sur un film Kodak-X Omat S et exposée pendant 4 à 6 jours à la température de -70°C. Les résultats sont représentés aux Figures 3, 4 et 5 annexées.

   La Figure 3 montre une immunoélectrophorèse en fusée croisée à travers un gel intermédiaire, de préparations d'antigènes HSV-1 et HSV-2 fractionnées. La bande (a) correspond à une fraction contenant l'antigène spécifique du type HSV-2. Les anticorps présents dans le gel intermédiaire $G_i$ proviennent de sérum de lapin hyperimmun anti-HSV-1, tel que préparé dans l'exemple 2 (25 $\mu$l/cm$^2$) et dans le gel de référence $G_r$, on trouve le sérum de lapin hyperimmun anti-HSV-2 (15 $\mu$l/cm$^2$). La bande (b) correspond à la fraction contenant l'antigène spécifique du type HSV-1. Les anticorps présents dans le gel intermédiaire $G_i$ proviennent de sérum de lapin hyperimmun anti-HSV-2 (25 $\mu$l/cm$^2$) et dans le gel de référence $G_r$, on trouve du sérum de lapin hyperimmun anti-HSV-1 (15 $\mu$l/cm$^2$). Les gels sont colorés par du Bleu brillant de Coomassie.

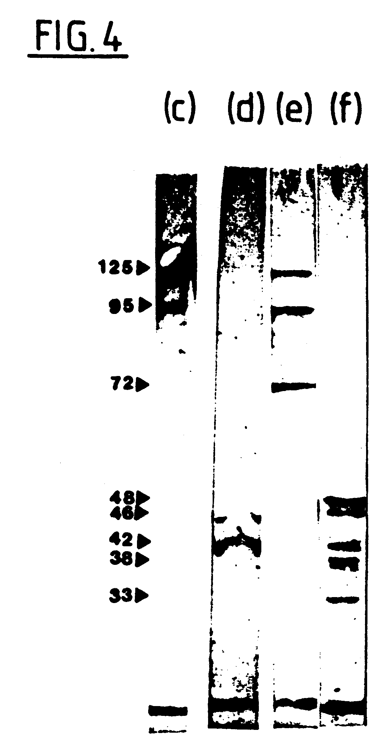

   La Figure 4 montre un autoradiogramme de SDS-PAGE. Les peptides présents dans les solutions d'antigènes viraux sont analysés par immunoélectrophorèse en fusée croisée et solubilisation. Dans la bande (c), 20 $\mu$l d'antigène spécifique HSV-1 marqué à la $^{35}$S-méthionine ont précipité dans le gel de référence contenant 15 $\mu$l/cm$^2$ d'anticorps de lapin anti-HSV-1 ; dans la bande (d), 20 $\mu$l d'antigène spécifique HSV-2 marqué par la $^{35}$S-méthionine ont précipité dans le gel de référence contenant 15 $\mu$l/cm$^2$ d'anticorps de lapin anti-HSV-2. Puis 10 $\mu$l de chaque fraction contenant les antigènes spécifiques du type HSV-1 ou HSV-2 sont soumis à une électrophorèse SDS-PAGE en l'absence d'antisérum et les bandes (e) et (f) montrent les résultats obtenus. Les poids moléculaires des protéines sont indiqués en kilodaltons à gauche des quatre bandes représentées à la Figure 4.

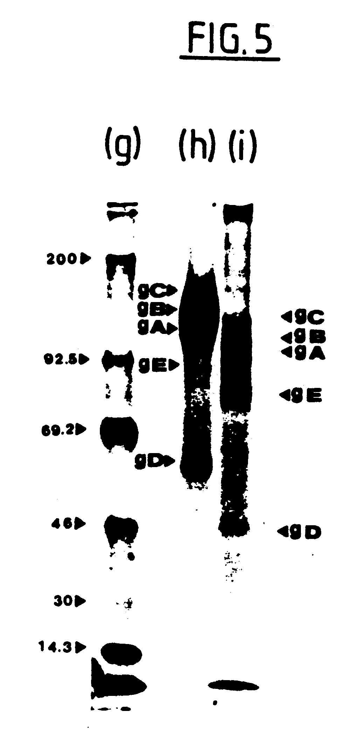

   La Figure 5 représente un autoradiogramme de glycoprotéines, marquées comme indiqué au début du présent exemple, séparées par électrophorèse, provenant de cellules RL infectées par HSV-1 dans le cas de la bande (h) et par HSV-2 dans le cas de la bande (i) ; la bande (g) correspond aux marqueurs de poids moléculaire, qui sont des protéines marquées au carbone 14. Les poids moléculaires des protéines sont indiqués en kilodaltons à gauche des trois bandes de cette Figure.

   Les polypeptides viraux ayant précipité [ Figure 4 , bandes (c) et (d)],sont identifiés par leurs poids moléculaires estimés par migration électrophorétique par rapport aux protéines standards marqueurs de poids moléculaire. Le polypeptide de 125 000 daltons, bande (c), est identifié comme correspondant à la glycoprotéine gC de HSV-1 (Spear, 1976, Journal of Virology 17:991-1008). Le polypeptide de 95 000 daltons que l'on voit également sur cette bande représente probablement un précurseur de la glycoprotéine gC, qui est également précipité dans l'immunoélectrophorèse en fusée croisée, par des sérums hyperimmuns anti-HSV-1 de lapin, du fait de l'existence de déterminants antigéniques communs. Le polypeptide de 46 000 daltons, bande (d), est identifié comme correspondant à la glycoprotéine gD de HSV-2, et pour les mêmes raisons que ci-dessus, on trouve un polypeptide de 42 000 daltons qui est probablement un précurseur de la glycoprotéine gD. Les bandes (e) et (f) de la Figure 4 montrent que les fractions contenant les antigènes spécifiques des types comprennent aussi trois ou quatre antigènes communs aux deux types de virus. Le radiomarquage des glycoprotéines virales par la D-(1-$^{14}$C)-glucosamine et l'autoradiographie des glycoprotéines séparées par électrophorèse (Figure 5) montrent effectivement que la glycoprotéine gD de HSV-2 (bande i) a un poids moléculaire de 46 000 daltons et que la glycoprotéine gC de HSV-1 (bande h) a un poids moléculaire de 125 000 daltons.

**EXEMPLE 5 :** Correspondance entre les polypeptides de 46 000 et 125 000 daltons et les glycoprotéines gC de HSV-1 et gD de HSV-2.

   Pour prouver que les polypeptides de 46 000 et 125 000 daltons correspondent bien respectivement aux glycoprotéines gD de HSV-2 et gC de HSV-1, on a procédé comme suit :

   - Production d'antisérum monospécifique.

Les parties supérieures des immunoprécipités obtenus à l'exemple 4 dans le gel de référence [Figure 3, bandes (a) et (b)], contenant les antigènes spécifiques des types, sont prélevées du gel. Lesdits précipités isolés sont désagrégés par ultrasons (20 kc/sec., deux fois sur de la glace pendant 30 secondes) puis mélangés volume à volume avec de l'adjuvant incomplet de Freund, puis injectés à des lapins, à quatre reprises à des intervalles de deux semaines entre chaque injection (Vestergaard, Norrild, 1978, Journal of Infectious Diseases 138:639-643). Les immunoglobulines obtenues sont purifiées et leur concentration en protéines ajustée à 30 mg/ml.

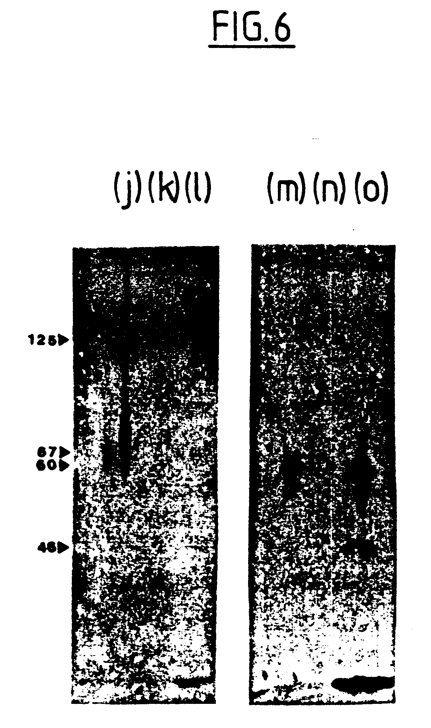

- Les polypeptides recueillis à partir des cellules infectées par HSV-1, par HSV-2, ainsi que des cellules témoins, sont séparés par électrophorèse sur gel de polyacrylamide à 7,5 %, comme décrit dans l'exemple 4, puis, les protéines sont transférées sur une feuille de nitrocellulose (dimension des pores : 0,22 m) à 220 mA et à 60 V pendant 2 heures, en utilisant une cellule de transfert Bio-Rad (Towbin et al, 1979, Proceedings of the National Academy of Science, USA 79:4350-4354). Avant de faire réagir les protéines avec les antisérums monospécifiques correspondants, les feuilles de nitrocellulose sont bloquées par du PBS contenant 3 % de sérumalbumine bovine. Après réaction des antisérums monospécifiques de lapin avec la feuille de nitrocollulose (dilution (1/400), les anticorps fixés sont détectés à l'aide de protéine A marquée à l'iode 125 et autoradiographiés pendant 16 heures à -70°C. On utilise la protéine A du staphylocoque doré (Pharmacia Fine Chemicals), marquée par de l'iode 125 (Amersham) par la méthode à la lactopéroxydase (Marchalonis, 1969, Biochemistry Journal 113: 299-305). Les résultats sont représentés à la Figure 6 annexée.

La Figure 6 montre la réaction, après transfert sur la feuille de nitrocellulose, entre les polypeptides de HSV-1, de HSV-2 et de cellules témoins et, d'une part l'antisérum monospécifique anti-polypeptide de 125 000 daltons, bandes (j), (k), et (l), et d'autre part l'antisérum monospécifique anti-polypeptide de 46 000 daltons, bandes (m), (n) et (o). Les bandes (j) et (m) correspondent aux polypeptides de HSV-1, les bandes (k) et (n) correspondent aux polypeptides des cellules témoins, et les bandes (l) et (o) aux polypeptides de HSV-2. Les poids moléculaires sont donnés à gauche des bandes, en kilodaltons.

Les bandes (j) et (l) correspondent à la réaction entre les polypeptides de HSV-1 et de HSV-2 respectivement, et l'antisérum monospécifique anti-polypeptide de 125 000 daltons. Ce sérum réagit fortement avec le polypeptide de 125 000 daltons de HSV-1, comme le montre la bande (j), et faiblement avec un polypeptide de 67 000 daltons commun à HSV-1 et HSV-2, comme le montre la bande (l). La dénaturation de certains déterminants antigéniques en présence du SDS pourrait contribuer à la détection du polypeptide de 67 000 daltons commun à HSV-1 et HSV-2 par l'antisérum monospécifique anti-HSV-1.

Les bandes (m) et (o) correspondent à la réaction entre les polypeptides de HSV-1 et HSV-2 respectivement et l'antisérum monospécifique anti-polypeptide de 46 000 daltons. La réaction la plus forte est observée dans ce cas avec le polypeptide de 46 000 daltons de HSV-2, comme le montre la bande (o), et une faible réaction est observée avec un polypeptide de 60 000 daltons comme le montre la bande (m). Les polypeptides provenant des cellules témoins, comme le montrent les bandes (k) et (n),ne réagissent avec aucun des antisérums monospécifiques.

Il est ainsi démontré que les polypeptides de 125 000 et 46 000 daltons sont identifiés comme étant respectivement la glycoprotéine gC de HSV-1 et la glycoprotéine gD de HSV-2.

**EXEMPLE 6 :** Détection des anticorps spécifiques des types HSV-1 et HSV-2 dans les liquides biologiques à l'aide des fractions conformes à l'invention, par la technique ELISA.

- Conditions de mise en oeuvre de la méthode ELISA.

La détermination des anticorps-immunoglobulines G (IgG) anti-HSV-1 et anti-HSV-2 dans le sérum humain, est réalisée sur des plaques de microtitration en polystyrène (Plaques Immunologiques, Nunc, 1-96F, Danemark). Les antigènes spécifiques de chacun des deux types, dilués dans du tampon carbonate 0,05 M (pH 9,6), sont respectivement fixés sur les plaques par incubation à 4°C pendant toute une nuit. Ces plaques sont ensuite lavées deux fois dans du PBS-Tween® 20 (0,05 %), et 100 $\mu$l de PBS-Tween®20 (0,05 %) contenant 1 % de sérumalbumine bovine sont ajoutés à chaque puits afin d'éviter les liaisons non spécifiques. Les plaques sont incubées pendant une heure à 37°C, lavées à deux reprises avec du PBS-Tween®20 (0,05 %), puis on ajoute à chaque puits 120 $\mu$l de sérum dilué dans du PBS-Tween®20 (0,05 %) contenant 1 % de sérumalbumine bovine (les dilutions de sérum successives sont multipliées par 2, et vont de 1:150 à 1:19200). Chaque plaque comprend des séries de puits contenant des sérums témoins positifs et négatifs. Les plaques sont incubées à 37°C pendant deux heures dans une chambre en atmosphère humide et lavées quatre fois dans du PBS-Tween®20 comme précédemment.

Une immunoglobuline IgG anti-globuline humaine est conjuguée à de la péroxydase de raifort spécifique des chaines $\gamma$ (Dako, Danemark), et diluée au 1:1000 dans du PBS-Tween® 20 contenant 1 % de

EP 0 263 025 B1

sérumalbumine bovine. On ajoute 120 $\mu$l de cet antisérum conjugué à chaque puits et on incube à 37°C pendant 1 heure en chambre humide. Après avoir lavé les plaques quatre fois dans du PBS-Tween® 20, on ajoute à chaque puits 200 $\mu$l de solution de substrat (10 mg d'o-phénylènediamine, 0,03 % d'$H_2O_2$ ajoutée immédiatement avant l'emploi dans 100 ml de tampon de substrat à pH 5 contenant 25,7 ml de phosphate disodique 0,2 M, 24,3 ml d'acide citrique 0,1 M, et 50 ml d'eau désionisée). La réaction est achevée par ajout de 50 $\mu$l par puits d'HCl 3N au bout de 20 minutes. On mesure ensuite l'absorbance à une longueur d'onde de 492 nm sur un appareil Titertek Multiskan (Laboratoires Flow, Angleterre).

Les sérums témoins négatifs sont utilisés pour déterminer une valeur limite, définie code l'absorbance spécifique moyenne des sérums témoins négatifs augmentée de 3 déviations standards à partir de cette moyenne. Tout sérum à absorbance spécifique supérieure à cette valeur limite est considéré comme positif pour les anticorps anti-virus.

- Examen de liquides biologiques provenant de patients humains.

Trois groupes de patients ont été examinés.

a) 1er groupe : des échantillons de sérum provenant de 328 patients hospitalisés dans différents hôpitaux d'Athènes (âge moyen de 43 ans, de 7 à 68 ans).

b) 2ème groupe : des échantillons de liquide céphalo-rachidien (LCR) provenant de 92 patients présentant divers troubles neurologiques.

c) 3ème groupe : des échantillons de sérum provenant de 29 patients présentant un herpès génital récurrent (15 sérums) ou labial (14 sérums) reconnus par les méthodes usuelles.

En outre, des sérums ont été obtenus de 3 patients présentant une infection par le virus cytomégalique (CMV), 3 patients présentant une infection par le virus (VZV), et 3 patients présentant une infection par le virus d'Epstein-Barr (EBV), infections démontrées par une augmentation significative du titre d'anticorps par le test de fixation du complément (CMV, VZV) en utilisant des antigènes capables de fixer le complément (commercialisés par les Laboratoires Virion Diagnostic, Suisse) et par détection d'anticorps IgM anti-EBV. Le test de fixation du complément est réalisé selon la microtechnique de Casey (1965,DC:US public Health Service, p. 31-34). Les anticorps IgM anti-virus d'Epstein-Barr ont été déterminés par une technique d'immunofluorescence indirecte, à l'aide de cellules $P_3HR_1$ fixées sur des lames de microscope et des anticorps anti-IgM humain de chèvre, conjugués avec de l'isothiocyanate de fluoresceine à une dilution de 1:100 (Pasteur Diagnostic France).

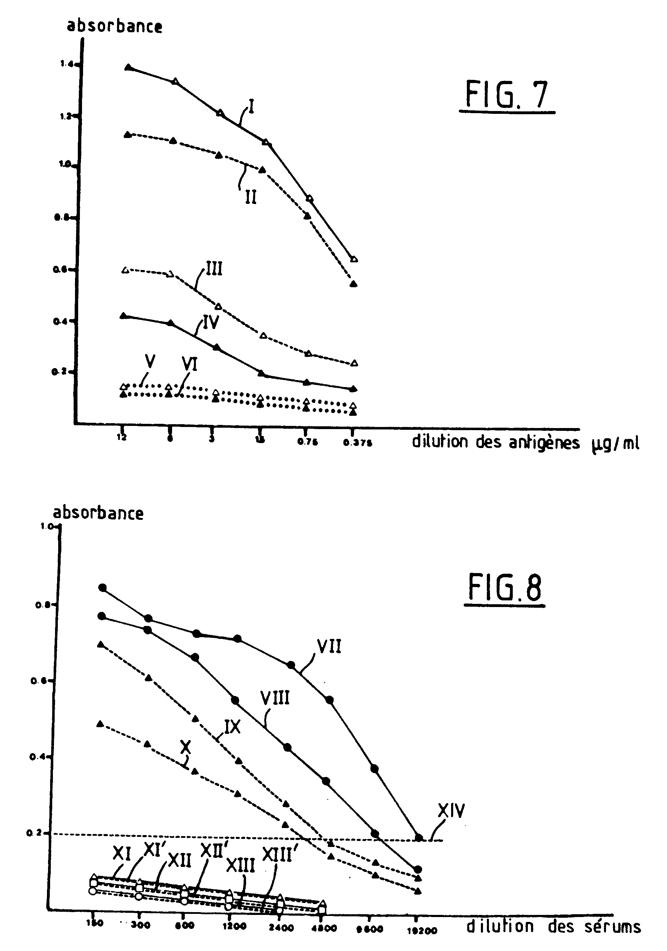

Les résultats obtenus avec les différents liquides biologiques tels que décrits ci-dessus sont représentés par des courbes (Figures 7 et 8 annexées).

La Figure 7 représente les courbes d'absorbance obtenues par réaction de fractions antigéniques HSV-1 et HSV-2 avec des immun-sérums : l'absorbance est donnée en ordonnée et les dilutions, en $\mu$g/ml, des fractions antigéniques spécifiques des types HSV-1 et HSV-2, sont données en abscisse.

Courbe I :       fraction antigénique HSV-1 + sérum anti-HSV-1
Courbe II :      fraction antigénique HSV-2 + sérum anti-HSV-2
Courbe III:      fraction antigénique HSV-1 + sérum anti-HSV-2
Courbe IV :      fraction antigénique HSV-2 + sérum anti-HSV-1
Courbe V :       fraction antigénique HSV-1 + sérum témoin négatif
Courbe VI :      fraction antigénique HSV-2 + sérum témoin négatif

La Figure 8 représente les courbes d'absorbance obtenues par réaction de fractions antigéniques HSV-1 et HSV-2 avec des immun-sérums : l'absorbance est donnée en ordonnée et les dilutions des immun-sérums utilisés, sont données en abscisse.

Courbe VII :      fraction antigénique HSV-1 + sérum anti-HSV-1
Courbe VIII:      fraction antigénique HSV-1 + sérum anti-HSV-1 différent de celui de la courbe VII
Courbe IX :       fraction antigénique HSV-2 + sérum anti-HSV-2
Courbe X :        fraction antigénique HSV-2 + sérum anti-HSV-2 différent de celui de la courbe IX
Courbe XI :       fraction antigénique HSV-1 + sérum anti-EBV
Courbe XI' :      fraction antigénique HSV-2 + sérum anti-EBV
Courbe XII :      fraction antigénique HSV-1 + sérum anti-VZV
Courbe XII' :     fraction antigénique HSV-2 + sérum anti-VZV
Courbe XIII :     fraction antigénique HSV-1 + sérum anti-CMV
Courbe XIII':     fraction antigénique HSV-2 + sérum anti-CMV
Courbe XIV :      valeur-limite.

Sur les microplaques où l'on a fixé à différentes dilutions les fractions contenant les antigènes spécifiques HSV-1 ou HSV-2 (dilutions de 6 à 0,375 $\mu$g de protéines/ml), on réalise une détection immunoenzymatique desdits antigènes par la méthode ELISA, avec une dilution constante (1:150) de sérums humains de référence HSV-1 et HSV-2 positifs. Les dilutions d'angigène qui conduisent aux plus

EP 0 263 025 B1

grands écarts entre le sérum de référence HSV-1 et HSV-2 sont déterminés à partir des courbes de dilutions standards (Figure 7). Pour cribler les sérums humains, on utilise une dilution d'antigène correspondant à 1,5 $\mu$g/ml aussi bien pour HSV-1 que pour HSV-2. Les courbes de la Figure 7 montrent la réactivité entre des fractions contenant les antigènes spécifiques de HSV-1 et HSV-2 avec les différents sérums employés quand on met en oeuvre la méthode ELISA.

Pour montrer la spécificité des fractions antigéniques par rapport aux anticorps anti-HSV-1 ou anti-HSV-2, on a mis en oeuvre la méthode ELISA avec 3 sérums anti-CMV, 3 sérums anti-VZV ou 3 sérums anti-EBV, en présence de fractions contenant les antigènes spécifiques HSV-1 et HSV-2 (Figure 8). Aucune réaction positive n'a été trouvée dans ces sérums, ce qui montre bien la spécificité des 2 fractions antigéniques pour le virus herpétique.

Un total de 328 sérums humains et de 92 liquides céphalo-rachidiens (LCR) a été testé par la méthode ELISA, la mesure de l'absorbance ayant été utilisée pour déterminer la présence ou l'absence d'anticorps anti-HSV-1 et anti-HSV-2 dans ces liquides biologiques. Un sérum est classé comme négatif pour les anticorps anti-virus si les valeurs de l'ab-sorbance (à 492 nm) sont inférieures à 0,2, et pour les LCR si ces valeurs d'absorbance sont inférieures à 0,15. Les patients qui sont infectés par HSV-1 ou par HSV-2 présentent à la fois des réponses aux antigènes homologues et hétérologues. Les patients infectés par HSV-1 présentent une forte réponse de type spécifique, ainsi le pourcentage de sérums positifs vis-à-vis de l'antigène hétérologue HSV-2 est de 4,5 % par rapport au nombre total de sérums positifs vis-à-vis de l'antigène homologue HSV-1, et le pourcentage de LCR positifs vis-à-vis de l'antigène hétérologue HSV-2 est de 0 % par rapport au nombre total de LCR positifs vis-à-vis de l'antigène homologue HSV-1 (les résultats sont représentés sur les Tableaux III et IV). Par contre, les patients infectés par HSV-2 présentent une très forte réponse de type commun en ce qui concerne les sérums, ainsi le pourcentage de sérums positifs vis-à-vis de l'antigène hétérologue HSV-1 est de 71,4 % par rapport au nombre total de sérums positifs vis-à-vis de l'antigène homologue HSV-2, cette réponse de type commun étant nulle en ce qui concerne les LCR (les résultats sont représentés sur les Tableaux III et IV).

La mise en oeuvre de la méthode ELISA avec 14 sérums humains, présentant une infection reconnue par HSV-1 et avec 15 sérums humains présentant une infection reconnue par HSV-2, en présence de préparations d'antigène commun de type HSV-1 et HSV-2 et en présence de fractions contenant les antigènes spécifiques HSV-1 et HSV-2, révèle que tous ces sérums réagissent contre les antigènes communs des types HSV-1 et HSV-2, ainsi que le montrent les Tableaux V et VI. Néanmoins, des titres positifs des sérums HSV-1 et HSV-2 sont observés uniquement en présence de fractions contenant les antigènes spécifiques HSV-1 et HSV-2 respectivement. Quatre sérums (28,5 %) de patients présentant une infection par HSV-1 contiennent aussi des anticorps contre les fractions spécifiques HSV-2. Onze sérums (73,3 %) de patients présentant une infection par HSV-2 contiennent également des anticorps contre les fractions spécifiques HSV-1. En outre, on remarque que les sérums de patients présentant une infection par HSV-1 et dont les anticorps réagissent avec les deux types d'antigènes montrent toujours des titres plus élevés contre les antigènes homologues HSV-1, alors que les sérums de patients présentant une infection par HSV-2 montrent une réponse de type commun plus développée et dans trois cas (20 %) des titres plus élevés sont obtenus contre les antigènes hétérologues HSV-1. On remarque ainsi, d'après les Tableaux V et VI, que les patients présentant une infection par HSV-1 montrent une forte réponse de type spécifique contre l'antigène homologue alors que les patients présentant une infection par HSV-2 montrent une forte réponse de type commune contre l'antigène hétérologue probablement due au fait que les personnes présentant une infection par HSV-2 ont souvent présenté auparavant une infection par HSV-1.

TABLEAU III

| Détection des anticorps anti-HSV-1 ou anti-HSV-2 par la méthode ELISA dans 328 sérums. | | |
|---|---|---|
| | Nombre de sérums positifs vis-à-vis de l'antigène homologue | Nombre de sérums positifs vis-à-vis de l'antigène hétérologue |
| Test pour les anticorps anti-HSV-1 | 224 | 10 |
| Test pour les anticorps anti-HSV-2 | 14 | 10 |

12

70 (21,4 %) sérums examinés sont négatifs vis-à-vis des deux antigènes. Les sérums sont examinés à des dilutions allant de 1:150 à 1:19 200.

TABLEAU IV

| Détection des anticorps anti-HSV-1 ou anti-HSV-2 par la méthode ELISA dans 92 LCR. | | |
|---|---|---|
| | Nombre de LCR positifs vis-à-vis de l'antigène homologue | Nombre de LCR positifs vis-à-vis de l'antigène hétérologue |
| Test pour les anticorps anti-HSV-1 | 36 | 0 |
| Test pour les anticorps anti-HSV-2 | 0 | 0 |

56 (60,9 %) LCR examinés sont négatifs vis-à-vis des deux antigènes. Les LCR sont examinés à des dilutions allant de 1:20 à 1:2 560.

TABLEAU V

| Titrages par la méthode ELISA de 14 sérums de patients présentant une infection par HSV-1 récurrente. | | | | |
|---|---|---|---|---|
| Titres [a] ELISA contre : | | | | |
| Patient No. | Antigène HSV-1 commun | Antigène HSV-2 commun | Fraction HSV-1 spécifique | Fraction HSV-2 spécifique |
| 1 | 1:4 800 | 1:1 200 | 1:4 800 | Neg[b] |
| 2 | 1:2 400 | 1:600 | 1:2 400 | Neg |
| 3 | 1:4 800 | 1:1 200 | 1:2 400 | Neg |
| 4 | 1:2 400 | 1:600 | 1:2 400 | Neg |
| 5 | 1:19 200 | 1:9 600 | 1:19 200 | 1:1 200 |
| 6 | 1:9 600 | 1:4 800 | 1:9 600 | Neg |
| 7 | 1:9 600 | 1:2 400 | 1:4 800 | Neg |
| 8 | 1:19 200 | 1:4 800 | 1:9 600 | 1:300 |
| 9 | 1:9 600 | 1:2 400 | 1:9 600 | 1:150 |
| 10 | 1:9 600 | 1:2 400 | 1:4 800 | Neg |
| 11 | 1:9 600 | 1:4 800 | 1:4 800 | 1:600 |
| 12 | 1:4 800 | 1:1 200 | 1:4 800 | Neg |
| 13 | 1:2 400 | 1:300 | 1:1 200 | Neg |
| 14 | 1:4 800 | 1:1 200 | 1:2 400 | Neg |

[a] Les sérums sont examinés à des dilutions allant de 1:150 à 1:19 200
[b] Le titre est négatif quand il est inférieur à 1:150 (chaque préparation antigénique est fixée sur les microplaques à des dilutions de 1,5 $\mu$g/ml).

TABLEAU VI

| Titrages par la méthode ELISA de 15 sérums de patients présentant une infection par HSV-2 récurrente. | | | | |
|---|---|---|---|---|
| | Titres[a] ELISA contre : | | | |
| Patient No. | Antigène HSV-1 commun | Antigène HSV-2 commun | Fraction HSV-1 spécifique | Fraction HSV-2 spécifique |
| 15 | 1:4 800 | 1:1 200 | 1:300 | 1:300 |
| 16 | 1:1 200 | 1:600 | Neg[b] | 1:300 |
| 17 | 1:2 400 | 1:1 200 | Neg | 1:300 |
| 18 | 1:2 400 | 1:600 | Neg | 1:150 |
| 19 | 1:19 200 | 1:9 600 | 1:2 400 | 1:4 800 |
| 20 | 1:9 600 | 1:9 600 | 1:1 200 | 1:2 400 |
| 21 | 1:19 200 | 1:9 600 | 1:2 400 | 1:4 800 |
| 22 | 1:9 600 | 1:9 600 | 1:150 | 1:2 400 |
| 23 | 1:4 800 | 1:4 800 | Neg | 1:2 400 |
| 24 | 1:9 600 | 1:4 800 | 1:1 200 | 1:600 |
| 25 | 1:19 200 | 1:9 600 | 1:2 400 | 1:1 200 |
| 26 | 1:4 800 | 1:4 800 | 1:1 200 | 1:1 200 |
| 27 | 1:9 600 | 1:4 800 | 1:2 400 | 1:2 400 |
| 28 | 1:19 200 | 1:9 600 | 1:4 800 | 1:2 400 |
| 29 | 1:4 800 | 1:2 400 | 1:1 200 | 1:1 200 |

[a] Les sérums sont examinés à des dilutions allant de 1:150 à 1:19 200.
[b] Le titre est négatif quand il est inférieur à 1:150 (chaque préparation antigénique est fixée sur les microplaques à des dilutions de 1,5 $\mu$g/ml).

**Revendications**
**Revendications pour les Etats contractants suivants : BE, CH, DE, FR, GB, IT, LI, LU, NL, SE**

1.   Procédé de purification de fractions antigéniques spécifiques de type de HSV-1 ou de HSV-2, dans lequel on procède à la multiplication in-vitro, sur des cellules de mammifère approprié, de cultures de virus HSV-1 et/ou HSV-2, jusqu'à la lyse d'au moins une proportion importante de cellules, puis on lyse les cellules restantes par des agents chimiques appropriés, et après désagrégation de leur paroi par traitement physique, notamment par ultrasons, on les soumet à une centrifugation, et l'on centrifuge le surnageant obtenu sur un gradient linéaire de saccharose, lequel procédé est caractérisé en ce que les antigènes spécifiques de HSV-1 et de HSV-2 sont respectivement recueillis dans les fractions du gradient contenant de 20 à 27% en poids de saccharose, et dans les fractions du gradient contenant de 6 à 10% en poids de saccharose.

2.   Fraction antigénique purifiée spécifique de type de HSV-1, caractérisée en ce qu'elle est susceptible d'être obtenue par le procédé selon la revendication 1, dans les fractions de 20 à 27% en poids d'un gradient linéaire de saccharose, et en ce que son coefficient de sédimentation est compris entre 9 et 9,6 S.

3.   Fraction antigénique purifiée spécifique de type de HSV-2, caractérisée en ce qu'elle est susceptible d'être obtenue par le procédé selon la revendication 1, dans les fractions de 6 à 10 % en poids d'un gradient linéaire de saccharose, et en ce que son coefficient de sédimentation est compris entre 4,3 et 4,5 S.

4.   Méthode de diagnostic des infections par le virus de l'herpès des types 1 ou 2, par détection d'anticorps anti-HSV-1 et/ou anti-HSV-2 dans un milieu biologique, caractérisée en ce que l'on met en contact une fraction antigénique spécifique de HSV-1 ou HSV-2 selon l'une quelconque des revendications 2 ou 3, fixée d'une manière appropriée sur un support solide approprié, avec le milieu biologique présumé contenir des anticorps anti-HSV-1 et/ou anti-HSV-2 examiné, en ce que l'immuncomplexe obtenu dans le cas où des anticorps anti-HSV-1 et/ou anti-HSV-2 sont effectivement présents, est détecté par un anticorps anti-espèce marqué par une enzyme, en présence d'un substrat spécifique de l'enzyme.

5. Méthode selon la revendication 4, caractérisée en ce que les fractions contenant les antigènes spécifiques HSV-1 et/ou HSV-2 sont fixées sur ledit support à des dilutions avantageusement comprises entre 0,375 et 6 $\mu$g de protéines par ml.

6. Méthode selon l'une quelconque des revendications 4 ou 5, caractérisée en ce que le support solide est choisi dans le groupe des supports comprenant notamment les microplaques, les tubes, les globules rouges, les billes de polystyrène et les feuilles de nitrocellulose.

7. Ensemble, ou kit, de diagnostic, pour la détermination, dans des liquides biologiques corporels, d'anticorps anti-HSV-1 et/ou anti-HSV-2, qui comprend comme composants principaux :
   - une quantité appropriée, éventuellement subdivisée en doses unitaires, de différentes dilutions de fraction antigénique spécifique de HSV-1 et de fraction antigénique spécifique de HSV-2 selon l'une quelconque des Revendications 2 ou 3,
   - une quantité appropriée, éventuellement subdivisée en doses unitaires, suffisante pour la détermination précédente, d'anticorps anti-espèce marqués par une enzyme, pour la révélation de la réaction antigène-anticorps entre l'anticorps à déterminer et l'antigène spécifique correspondant ;
   - éventuellement une quantité appropriée de tampons, diluants, réactifs, nécessaires à la mise en oeuvre de la méthode de diagnostic selon l'une quelconque des revendications 4 à 6.

8. Application des fractions antigéniques selon l'une quelconque des revendications 2 ou 3 au diagnostic différentiel *in vitro* de HSV-1 et de HSV-2

**Revendications pour les Etats contractants suivants : AT, ES, GR**

1. Procédé de purification de fractions antigéniques spécifiques de type de HSV-1 ou de HSV-2, dans lequel on procède à la multiplication in-vitro, sur des cellules de mammifère approprié, de cultures de virus HSV-1 et/ou HSV-2, jusqu'à la lyse d'au moins une proportion importante de cellules, puis on lyse les cellules restantes par des agents chimiques appropriés, et après désagrégation de leur paroi par traitement physique, notamment par ultrasons, on les soumet à une centrifugation, et l'on centrifuge le surnageant obtenu sur un gradient linéaire de saccharose, lequel procédé est caractérisé en ce que les antigènes spécifiques de HSV-1 et de HSV-2 sont respectivement recueillis dans les fractions du gradient contenant de 20 à 27% en poids de saccharose, et dans les fractions du gradient contenant de 6 à 10% en poids de saccharose.

2. Procédé selon la revendication 1, caractérisé en ce que la fraction antigénique spécifique de type de HSV-1 recueillie dans les fractions de 20 à 27% en poids d'un gradient linéaire de saccharose, possède un coefficient de sédimentation compris entre 9 et 9,6 S.

3. Procédé selon la revendication 1, caractérisé en ce que la fraction antigénique spécifique de type de HSV-2 recueillie dans les fractions de 6 à 10 % en poids d'un gradient linéaire de saccharose, possède un coefficient de sédimentation compris entre 4,3 et 4,5 S.

4. Méthode de diagnostic des infections par le virus de l'herpès des types 1 ou 2, par détection d'anticorps anti-HSV-1 et/ou anti-HSV-2 dans un milieu biologique, caractérisée en ce que l'on met en contact une fraction antigènique spécifique de HSV-1 ou HSV-2 obtenue par un procédé selon l'une quelconque des revendications 1 à 3, fixée d'une manière appropriée sur un support solide approprié, avec le milieu biologique présumé contenir des anticorps anti-HSV-1 et/ou anti-HSV-2 examiné, en ce que l'immun-complexe obtenu dans le cas où des anticorps anti-HSV-1 et/ou anti-HSV-2 sont effectivement présents, est détecté par un anticorps anti-espèce marqué par une enzyme, en présence d'un substrat spécifique de l'enzyme.

5. Méthode selon la revendication 4, caractérisée en ce que les fractions contenant les antigènes spécifiques HSV-1 et/ou HSV-2 sont fixées sur ledit support à des dilutions avantageusement comprises entre 0,375 et 6 $\mu$g de protéines par ml.

**6.** Méthode selon l'une quelconque des revendications 4 ou 5, caractérisée en ce que le support solide est choisi dans le groupe des supports comprenant notamment les microplaques, les tubes, les globules rouges, les billes de polystyrène et les feuilles de nitrocellulose.

**7.** Application des fractions antigéniques obtenues par un procédé selon l'une quelconque des revendications 1 à 3, au diagnostic différentiel *in vitro* de HSV-1 et de HSV-2

**Claims**
**Claims for the following Contracting States : BE, CH, DE, FR, GB, IT, LI, LU, NL, SE**

**1.** Process for the purification of specific antigenic fractions of HSV-1 or HSV-2 type, in which in-vitro proliferation, in cells of the appropriate mammal, of cultures of HSV-1 and/or HSV-2 virus is carried out until at least a significant proportion of cells have been lysed, then the remaining cells are lysed by appropriate chemical agents, and after disintegration of their wall by physical treatment, in particular by ultrasound, they are subjected to a centrifugation, and the supernatant obtained is centrifuged on a linear sucrose gradient, which process is characterised in that the specific HSV-1 and HSV-2 antigens are respectively collected in the fractions of the gradient containing from 20 to 27% by weight of sucrose, and in the fractions of the gradient containing from 6 to 10% by weight of sucrose.

**2.** Specific purified antigenic fraction of HSV-1 type, characterised in that it can be obtained by the process according to Claim 1, in the fractions from 20 to 27% by weight of a linear sucrose gradient, and in that its sedimentation coefficient is between 9 and 9.6 S.

**3.** Specific purified antigenic fraction of HSV-2 type, characterised in that it can be obtained by the process according to Claim 1, in the fractions from 6 to 10% by weight of a linear sucrose gradient, and in that its sedimentation coefficient is between 4.3 and 4.5 S.

**4.** Method of diagnosis of herpes virus type 1 or 2 infections, by detection of anti-HSV-1 and/or anti-HSV-2 antibodies in a biological medium, characterised in that a specific antigenic fraction of HSV-1 or HSV-2 according to either of Claims 2 and 3, immobilised in an appropriate manner on an appropriate solid support, is contacted with the biological medium examined which is presumed to contain anti-HSV-1 and/or anti-HSV-2 antibodies, and in that the immune complex obtained in the case in which anti-HSV-1 and/or anti-HSV-2 antibodies are indeed present is detected by an anti-species antibody labelled by an enzyme, in the presence of a specific substrate of the enzyme.

**5.** Method according to Claim 4, characterised in that the fractions containing the specific HSV-1 and/or HSV-2 antigens are immobilised on the said support at dilutions advantageously of between 0.375 and 6 $\mu$g of protein per ml.

**6.** Method according to either of Claims 4 and 5, characterised in that the solid support is chosen from amongst the group of supports comprising in particular microplates, tubes, red corpuscles, polystyrene beads and nitrocellulose sheets.

**7.** Diagnostic outfit or kit, for the determination, in corporeal biological fluids, of anti-HSV-1 and/or anti-HSV-2 antibodies, which comprises as principal components:
   - an appropriate quantity, optionally subdivided into unit doses, of different dilutions of specific antigenic fraction of HSV-1 and of specific antigenic fraction of HSV-2 according to either of Claims 2 and 3,
   - an appropriate quantity, optionally subdivided into unit doses, sufficient for the above determination, of anti-species antibodies labelled with an enzyme, for detecting the antigen-antibody reaction between the antibody to be determined and the corresponding specific antigen;
   - optionally an appropriate quantity of buffers, diluents or reagents necessary for implementing the diagnostic method according to any one of Claims 4 to 6.

**8.** Application of the antigenic fractions according to either of Claims 2 and 3 to the *in vitro* differential diagnosis of HSV-1 and HSV-2.

# EP 0 263 025 B1

**Claims for the following Contracting States : AT, ES, GR**

1. Process for the purification of specific antigenic fractions of HSV-1 or HSV-2 type, in which in-vitro proliferation, in cells of the appropriate mammal, of cultures of HSV-1 and/or HSV-2 virus is carried out until at least a significant proportion of cells have been lysed, then the remaining cells are lysed by appropriate chemical agents, and after disintegration of their wall by physical treatment, in particular by ultrasound, they are subjected to a centrifugation, and the supernatant obtained is centrifuged on a linear sucrose gradient, which process is characterised in that the specific HSV-1 and HSV-2 antigens are respectively collected in the fractions of the gradient containing from 20 to 27% by weight of sucrose, and in the fractions of the gradient containing from 6 to 10% by weight of sucrose.

2. Process according to Claim 1, characterised in that the specific antigenic fraction of HSV-1 type collected in the fractions from 20 to 27% by weight of a linear sucrose gradient has a sedimentation coefficient of between 9 and 9.6 S.

3. Process according to Claim 1, characterised in that the specific antigenic fraction of HSV-2 type collected in the fractions from 6 to 10% by weight of a linear sucrose gradient has a sedimentation coefficient of between 4.3 and 4.5 S.

4. Method of diagnosis of herpes virus type 1 or 2 infections, by detection of anti-HSV-1 and/or anti-HSV-2 antibodies in a biological medium, characterised in that a specific antigenic fraction of HSV-1 or HSV-2 obtained by a process according to any one of Claims 1 to 3, immobilised in an appropriate manner on an appropriate solid support, is contacted with the biological medium examined which is presumed to contain anti-HSV-1 and/or anti-HSV-2 antibodies, and in that the immune complex obtained in the case in which anti-HSV-1 and/or anti-HSV-2 antibodies are indeed present is detected by an anti-species antibody labelled by an enzyme, in the presence of a specific substrate of the enzyme.

5. Method according to Claim 4, characterised in that the fractions containing the specific HSV-1 and/or HSV-2 antigens are immobilized on the said support at dilutions advantageously of between 0.375 and 6 $\mu$g of protein per ml.

6. Method according to either of Claims 4 and 5, characterised in that the solid support is chosen from amongst the group of supports comprising in particular microplates, tubes, red corpuscles, polystyrene beads and nitrocellulose sheets.

7. Application of the antigenic fractions obtained by a process according to any one of Claims 1 to 3 to the *in vitro* differential diagnosis of HSV-1 and HSV-2.

**Patentansprüche**

**Patentansprüche für folgende Vertragsstaaten : BE, CH, DE, FR, GB, IT, LI, LU, NL, SE**

1. Verfahren zur Reinigung von spezifischen Antigenfraktionen des Typs HSV-1 oder HSV-2, wobei man in vitro auf geeigneten Säugerzellen Kulturen des HSV-1- und/oder HSV-2-Virus bis zur Lyse mindestens eines erheblichen Teils der Zellen vermehrt, anschließend die verbleibenden Zellen durch geeignete chemische Mittel lysiert und nach Zerteilung ihrer Zellwände durch physikalische Behandlung, insbesondere durch Ultraschall, sie zentrifugiert und den erhaltenen Überstand in einem linearen Saccharosegradienten zentrifugiert, dadurch **gekennzeichnet,** daß die spezifischen HSV-1- und HSV-2-Antigene jeweils aus den Fraktionen des Gradienten, die 20 bis 27 Gew. -% Saccharose enthalten, und aus den Fraktionen des Gradienten, die 6 bis 10 Gew. -% Saccharose enthalten, isoliert werden.

2. Spezifische gereinigte Antigenfraktion vom Typ HSV-1, dadurch **gekennzeichnet,** daß sie nach dem Verfahren nach Anspruch 1 aus Fraktionen mit 20 bis 27 Gew. -% eines linearen Saccharosegradienten erhalten werden kann und daß ihr Sedimentationskoeffizient zwischen 9 und 9,6 S liegt.

3. Spezifische gereinigte Antigenfraktion vom Typ HSV-2, dadurch **gekennzeichnet,** daß sie nach dem Verfahren nach Anspruch 1 aus Fraktionen mit 6 bis 10 Gew. -% eines linearen Saccharosegradienten erhalten werden kann und daß ihr Sedimentationskoeffizient zwischen 4,3 und 4,5 S liegt.

17

**4.** Verfahren zur Diagnose von Infektionen durch das Herpesvirus der Typen 1 oder 2 durch Detektion von Anti-HSV-1- und/oder Anti-HSV-2-Antikörpern in einem biologischen Milieu, dadurch **gekennzeichnet,** daß man eine spezifische Antigenfraktion von HSV-1 oder HSV-2 nach einem der Ansprüche 2 oder 3, welche in geeigneter Weise auf einen geeigneten festen Träger fixiert ist, mit dem untersuchten biologischen Milieu, das vermutlich Anti-HSV-1- und/oder HSV-2-Antikörper enthält, in Kontakt bringt, man den Immunkomplex, der im Falle, in dem Anti-HSV-1- und/oder Anti-HSV-2-Antikörper tatsächlich vorhanden sind, erhalten wird, durch einen antispeziesspezifischen Antikörper, der mit einem Enzym markiert ist, in Gegenwart eines spezifischen Enzymsubstrats detektiert.

**5.** Verfahren nach Anspruch 4, dadurch **gekennzeichnet,** daß die Fraktionen, die die spezifischen HSV-1- und/oder HSV-2-Antigene enthalten, auf den Träger in Verdünnungen, die vorteilhafterweise zwischen 0,375 und 6 µg Protein pro ml betragen, fixiert sind.

**6.** Verfahren nach einem der Ansprüche 4 oder 5, dadurch **gekennzeichnet,** daß der feste Träger aus der Gruppe der Träger, umfassend insbesondere Mikrotiterplatten, Reagenzgläser, rote Blutkörperchen, Polystyrolkugeln und Nitrocellulosefolien, ausgewählt ist.

**7.** Reagenszusammenstellung oder Kit zur Diagnose, um in biologischen Körperflüssigkeiten Anti-HSV-1- und/oder HSV-2-Antikörper zu bestimmen, umfassend als Hauptbestandteile:
- ein geeignete Menge, gegebenenfalls in Einzeldosen aufgeteilt, verschiedener Verdünnungen einer spezifischen HSV-1-Antigenfraktion und einer spezifischen HSV-2-Antigenfraktion nach einem der Ansprüche 2 oder 3,
- eine geeignete Menge, gegebenenfalls in Einzeldosen aufgeteilt, die für die vorstehende Bestimmung ausreicht, an antispeziesspezifischen Antikörpern, die durch ein Enzym markiert sind, zum Nachweis der Antigen/Antikörperreaktion zwischen dem zu bestimmenden Antikörper und dem entsprechenden spezifischen Antigen,
- gegebenenfalls eine geeignete Menge an Puffern, Verdünnungsmitteln, Reagentien, die zur Durchführung des Diagnoseverfahrens nach einem der Ansprüche 4 bis 6 notwendig sind.

**8.** Verwendung der Antigenfraktionen nach einem der Ansprüche 2 oder 3 zur in vitro-Differenzialdiagnose von HSV-1 und HSV-2.

**Patentansprüche für folgende Vertragsstaaten : AT, ES, GR**

**1.** Verfahren zur Reinigung von spezifischen Antigenfraktionen des Typs HSV-1 oder HSV-2, wobei man in vitro auf geeigneten Säugerzellen Kulturen des HSV-1- und/oder HSV-2-Virus bis zur Lyse mindestens eines erheblichen Teils der Zellen vermehrt, anschließend die verbleibenden Zellen durch geeignete chemische Mittel lysiert und nach Zerteilung ihrer Zellwände durch physikalische Behandlung, insbesondere durch Ultraschall, sie zentrifugiert und den erhaltenen Überstand in einem linearen Saccharosegradienten zentrifugiert, dadurch **gekennzeichnet,** daß die spezifischen HSV-1- und HSV-2-Antigene jeweils aus den Fraktionen des Gradienten, die 20 bis 27 Gew.-% Saccharose enthalten, und aus den Fraktionen des Gradienten, die 6 bis 10 Gew.-% Saccharose enthalten, isoliert werden.

**2.** Verfahren nach Anspruch 1, dadurch **gekennzeichnet,** daß die spezifische Antigenfraktion vom HSV-1-Typ, die aus den Fraktionen mit 20 bis 27 Gew.-% eines linearen Saccharosegradienten isoliert wird, einen Sedimentationskoeffizienten zwischen 9 und 9,6 S besitzt.

**3.** Verfahren nach Anspruch 1, dadurch **gekennzeichnet,** daß die spezifische Antigenfraktion vom HSV-2-Typ, die aus den Fraktionen mit 6 bis 10 Gew.-% eines linearen Saccharosegradienten isoliert wird, einen Sedimentationskoeffizienten zwischen 4,3 und 4,5 S besitzt.

**4.** Verfahren zur Diagnose von Infektionen durch das Herpesvirus der Typen 1 oder 2 durch Nachweis von Anti-HSV-1-und/oder und/oder Anti-HSV-2-Antikörpern in einem biologischen Milieu, dadurch **gekennzeichnet,** daß man eine spezifische Antigenfraktion von HSV-1 oder HSV-2, die nach einem Verfahren nach einem der Ansprüche 1 bis 3 erhalten wurde und in geeigneter Weise auf einem festen Träger fixiert ist, mit einem untersuchten biologischen Milieu, das vermutlich Anti-HSV-1-und/oder Anti-HSV-2-Antikörper enthält, in Kontakt bringt, man den Immunkomplex, der im Falle in dem Anti-HSV-1-und/oder Anti-HSV-2-Antikörper tatsächlich vorhanden sind, erhalten wird, durch einen antispeziesspezifischen Antikörper, der mit einem Enzym markiert ist, in Gegenwart eines für das Enzym spezifischen Substrats detektiert.

5. Verfahren nach Anspruch 4, dadurch **gekennzeichnet,** daß die Fraktionen, die die spezifischen HSV-1- und/oder HSV-2-Antigene enthalten, auf dem Träger in Verdünnungen, die vorteilhafterweise zwischen 0,375 und 6 $\mu$g Protein pro ml betragen, fixiert sind.

6. Verfahren nach einem der Ansprüche 4 oder 5, dadurch **gekennzeichnet,** daß der feste Träger aus der Gruppe der Träger, umfassend insbesondere Mikrotiterplatten, Reagenzgläser, rote Blutkörperchen, Polystyrolkugeln und Nitrocellulosefolien, ausgewählt ist.

7. Verwendung von Antigenfraktionen, erhalten nach einem Verfahren nach einem der Ansprüche 1 bis 3, zur in vitro-Differenzialdiagnose von HSV-1 und HSV-2.

# FIG. 1

# FIG. 2

EP 0 263 025 B1

# FIG.3

GR

GR

GI

GI

22

# FIG.4

(c)   (d) (e) (f)

125▶

95▶

72▶

48▶
46▶

42▶
38▶

33▶

# FIG.5

# FIG.6

(j) (k) (l)     (m) (n) (o)

125▷

67▷
60▷

46▷

FIG. 7

FIG.8